# EUROPÄISCHE PATENTANMELDUNG

(11) **EP 2 389 907 A1**
(43) Veröffentlichungstag der Anmeldung: **30.11.2011**
(21) Anmeldenummer: 10163783.3
(22) Anmeldetag: 25.05.2010
(51) Int. Cl.: A61F 5/01

(54) **Sprunggelenkbandage**

(71) Anmelder: Darco (Europe) GmbH, 82399 Raisting (DE)
(72) Erfinder: Dietrich, Thomas, 82399 Raisting (DE)
(74) Vertreter: Rupprecht, Kay

(57) **Zusammenfassung**

Sprunggelenkbandage zur Behandlung von Verletzungen des Sprunggelenks, mit einem unteren Basiselement (2) und einem Oberteil (4), welches über eine Gelenkverbindung (6) mit dem unteren Basiselement (2) in Wirkverbindung steht, wobei das untere Basiselement (2) ein Bodenelement (8), ein inneres aufragendes Stützelement (10) und ein äußeres aufragendes Stützelement (12) umfasst, welche miteinander verbunden sind, und wobei das äußere aufragende Stützelement (12) einen unteren Abschnitt (14) aufweist, der sich unterhalb des Außenknöchels befindet, und einen oberen Abschnitt (16), der sich oberhalb des Außenknöchels befindet.

Mit dem Ziel, ein Sprunggelenk optimal zu stabilisieren und eine physiologisch korrekte Passform einer Sprunggelenkbandage zu erreichen, ist vorgesehen, dass der untere Abschnitt (14) des äußeren aufragenden Stützelements (12) zur Fußlängsachse (A_{FL}) um einen Winkel (α) distal nach außen gedreht ist, und der obere Abschnitt (16) des äußeren aufragenden Stützelements (12) parallel zur Fußlängsachse (A_{FL}) angeordnet ist.

## Beschreibung

Die Erfindung betrifft eine Sprunggelenkbandage zur Behandlung von Verletzungen des Sprunggelenks, mit einem unteren Basiselement und einem Oberteil, welches über eine Gelenkverbindung mit dem unteren Basiselement in Wirkverbindung steht, wobei das untere Basiselement ein Bodenelement, ein inneres aufragendes Stützelement und ein äußeres aufragendes Stützelement umfasst, welche miteinander verbunden sind, und wobei das äußere aufragende Stützelement einen unteren Abschnitt aufweist, der sich unterhalb des Außenknöchels befindet, und einen oberen Abschnitt, der sich oberhalb des Außenknöchels befindet.

Solche Sprunggelenkbandagen sind aus dem Stand der Technik bekannt.

Aus der EP 1 152 720 B1 ist eine Sprunggelenkstütze mit Manschette bekannt, die Flexibilität bei Bewegungen in Vorwärts-Rückwärts-Richtung bei gleichzeitiger Verhinderung der seitlichen Bewegung des Knöchels bietet. Die Knöchelschiene umfasst schwenkbar verbundene Arme, die ein Einstellen der Schiene auf eine Vielzahl unterschiedlicher Füße und Beine von Menschen ermöglicht und eine Manschette ausbilden. Es ist ein Fersensteg vorgesehen, der die Ferse umgibt, und eine Zunge, die sich vom Fersensteg nach vorne erstreckt. Weiterhin umfasst die Schiene ein Sohlenteil, vertikale Seitenteile und Schwenkschenkel, die schwenkbar mit den Seitenteilen verbunden sind. Eine genaue Anpassung an die Form des Sprunggelenks ist jedoch nicht vorgesehen.

Die US 5,031,607 A beschreibt eine Sprunggelenkstütze, die ebenso ein Beugen des Knöchels nach vorne und nach hinten, also eine Plantarflexion und eine Dorsiflexion, gewährleistet, gleichzeitig jedoch eine Bewegung des Knöchels nach innen oder nach außen, also eine Inversion und eine Eversion, verhindert. Diese Sprunggelenkstütze besteht aus einem Fersenbügel, zwei Schwenklaschen und zwei Übungseinlagen oder Kompressionseinlagen. Diese Übungseinlagen beziehungsweise Kompressionseinlagen sind mit Vertiefungen versehen und können mit Befestigungsmitteln austauschbar an den Schwenklaschen angebracht werden. Die Gelenke, die den Fersenbügel mit den Schwenklaschen verbinden, also die Schwenkpunkte, liegen jedoch auf einer unterschiedlichen Höhe. Diese Art von Knöchelstütze ist am linken Fuß und am rechten Fuß tragbar. Eine genaue Anpassung an die Form des Innenknöchels (Malleolus medialis) und des Außenknöchels (Malleolus lateralis) ist jedoch außer einer Vertiefung für den jeweiligen Knöchel nicht vorgesehen.

Der Erfindung liegt die Aufgabe zugrunde, eine Sprunggelenkbandage der bisher bekannten Art derart weiterzubilden, dass bei hohem Tragekomfort ein Umbiegen des Knöchels nach innen und nach außen verhindert wird, um eine posttraumatische Verletzung zu behandeln oder präventiv die Stabilität des Sprunggelenks zu optimieren und zu garantieren. Gleichzeitig soll eine richtig angepasste Form an den Außenknöchel gewährleistet werden und dennoch die Möglichkeit einer Vorwärtsbewegung und einer Rückwärtsbewegung des Fußes bestehen.

Diese Aufgabe wird durch eine Sprunggelenkbandage der eingangs genannten Art dadurch gelöst, dass der untere Abschnitt des äußeren aufragenden Stützelements zur Fußlängsachse um einen Winkel distal nach außen gedreht ist, und der obere Abschnitt des äußeren aufragenden Stützelements parallel zur Fußlängsachse angeordnet ist.

Insgesamt wird erfindungsgemäß also eine Sprunggelenkbandage zur Behandlung von Verletzungen des Sprunggelenks angegeben, die im Wesentlichen aus einem unteren Basiselement und einem Oberteil besteht, die beide über eine Gelenkverbindung miteinander mechanisch kommunizieren. Diese Gelenkverbindung ermöglicht eine Schwenkbewegung des Oberteils gegenüber dem unteren Basiselement in Richtung der Fußlängsachse. Das untere Basiselement weist ein Bodenelement sowie ein inneres aufragendes Stützelement und ein äußeres aufragendes Stützelement auf, welche miteinander verbunden sind. Dabei besitzt das äußere aufragende Stützelement einen unteren Abschnitt, der sich unterhalb des Außenknöchels befindet, und einen oberen Abschnitt, der sich oberhalb des Außenknöchels befindet. Dabei ist der untere Abschnitt des äußeren aufragenden Stützelements zur Fußlängsachse um einen Winkel distal nach außen gedreht, und der obere Abschnitt des äußeren aufragenden Stützelements parallel zur Fußlängsachse angeordnet.

Die Fußlängsachse beziehungsweise sagittale Achse ist dabei die Achse, die in der Mitte entlang des Fußes horizontal verläuft. Das äußere aufragende Stützelement verläuft im Wesentlichen, mit Ausnahme des unteren Abschnitts, entlang einer parallel zu dieser Fußlängsachse verschobenen Achse. Eine Senkrechte zu dieser parallel verschobenen Achse entspricht der Längsachse des äußeren aufragenden Stützelements, das an der Außenkante des Fußes angelegt wird und sich von dort nach oben entlang der Wade erstreckt. Dabei wird der untere Abschnitt des äußeren aufragenden Stützelements, der unterhalb des Außenknöchels liegt, um diese Senkrechte gedreht. Dies erfolgt distal, also vom Außenknöchel des Fußes weg. Der obere Abschnitt des äußeren aufragenden Stützelements ist wieder in die Gegenrichtung des unteren Abschnitts gedreht, sodass er wieder parallel zur Fußlängsachse verläuft.

Dies bietet den Vorteil, dass das Sprunggelenk und insbesondere der verletzte Außenknöchel an einem Stützelement anliegt, dass eine an die anatomische Form des Fußes besonders angepasste Form aufweist. Dies stabilisiert den Außenknöchel optimal und verhindert so eine Bewegung, die der Verletzung schadet und sie somit verschlimmern würde. Die Mobilität des Fußes des Patienten wird jedoch nicht ganz eingeschränkt, sondern gezielt unterstützt, sodass krankengymnastische Übungen zur Heilung der Verletzung und eine Prävention einer neuen Verletzung gewährleistet werden können.

Ein wesentlicher Punkt der Erfindung liegt darin, dass die erfindungsgemässe Sprunggelenkbandage zur Behandlung von Verletzungen des Sprunggelenks derart ausgebildet ist, dass eine Stabilisierung des Sprunggelenks, und insbesondere des Knöchels garantiert wird, ohne die Möglichkeit krankengymnastischer Übungen und einer Behandlung und Heilung der Verletzung einzuschränken. Darüber hinaus werden normale Fußbewegungen, wie beispielsweise eine freie Vorwärtsbewegung (Plantarflexion) und eine freie Rückwärtsbewegung (Dorsalflexion), während der Verletzungszeit bzw. der Rehabilitierungszeit ermöglicht. Die Mobilität des Fußes des Patienten wird somit nur bedingt, jedoch gezielt durch das Verhindern eines Umbiegens des verletzten oder verletzungsgefährdeten Sprunggelenks eingeschränkt.

Bevorzugte Weiterbildungen der Erfindung ergeben sich aus den Unteransprüchen.

Vorzugsweise sind das Bodenelement, das innere aufragende Stützelement und das äußere aufragende Stützelement einstückig miteinander verbunden. Da dieses untere Basiselement aus Kunststoff besteht, kann dadurch eine kostengünstige und einfache Herstellung des unteren Basiselements mittels einer Spritzgussmaschine erzielt werden.

Um ein bequemes Tragen der Sprunggelenkbandage zu gewährleisten, weist der untere Abschnitt des äußeren aufragenden Stützelements vorzugsweise eine distal nach außen gerichtete Auswölbung auf. Es ist ja allgemein bekannt, dass der Mittelfuß beim Auftreten des Fußes unter einer Gehbewegung gespreizt wird, was bedeutet, dass er sich plantar, also auf Seiten der Planta pedis, vergrößert. Dies wird bei der erfindungsgemäßen Sprunggelenkbandage in sehr vorteilhafter Weise berücksichtigt. Der Mittelfuß hat somit die Möglichkeit, sich auszubreiten und sich in diese Auswölbung einzupassen, was zur zusätzlichen Stabilisierung des Knöchels beiträgt.

In bevorzugter Weise besitzt die Gelenkverbindung ein erstes Gelenk am oberen Ende des inneren aufragenden Stützelements und ein zweites Gelenk am oberen Ende des äußeren aufragenden Stützelements. Über diese Gelenkverbindung steht das untere Basiselement mit dem Oberteil der Sprunggelenkbandage in Wirkverbindung. Dies geschieht beispielsweise über eine Nietverbindung oder dergleichen bewegliche Befestigungsverbindung.

Vorzugsweise verläuft die durch das erste Gelenk und das zweite Gelenk verlaufende Achse horizontal oder nahezu horizontal. Dies stellt die mechanisch richtig liegende Anordnung der Drehgelenke dar und fördert so die physiologisch korrekte Fußbewegung. Somit wird eine zusätzliche Belastung des Sprunggelenks und insbesondere des Knöchels durch eine falsche Fußbewegung verhindert.

Schließlich ist für den Winkel, um den der untere Abschnitt des äußeren aufragenden Stützelements im Verhältnis zur Fußlängsachse distal nach außen gedreht ist, ein Bereich von 3,5° bis 7,0° vorgesehen. Eine besondere Anpassung an die anatomische Form des Fußes wird mit einem Winkelmass von 5,2° erreicht.

Nachfolgend wir die Erfindung anhand eines Ausführungsbeispiels beschrieben, welches in den folgenden Abbildungen näher erläutert wird. Hierbei zeigen:
- Fig. 1: eine perspektivische Darstellung einer Sprunggelenkbandage;
- Fig. 2: eine perspektivische Darstellung eines unteren Basiselements der Sprunggelenkbandage aus Fig. 1;
- Fig. 3: eine Frontansicht des unteren Basiselements aus Fig. 2; und
- Fig. 4: eine Seitenansicht einer gebrauchsfertigen Sprunggelenkbandage aus Fig. 1.

In der nachfolgenden Beschreibung werden für gleiche und gleich wirkende Teile dieselben Bezugsziffern verwendet, wobei bisweilen Hochindizes ihre Anwendung finden.

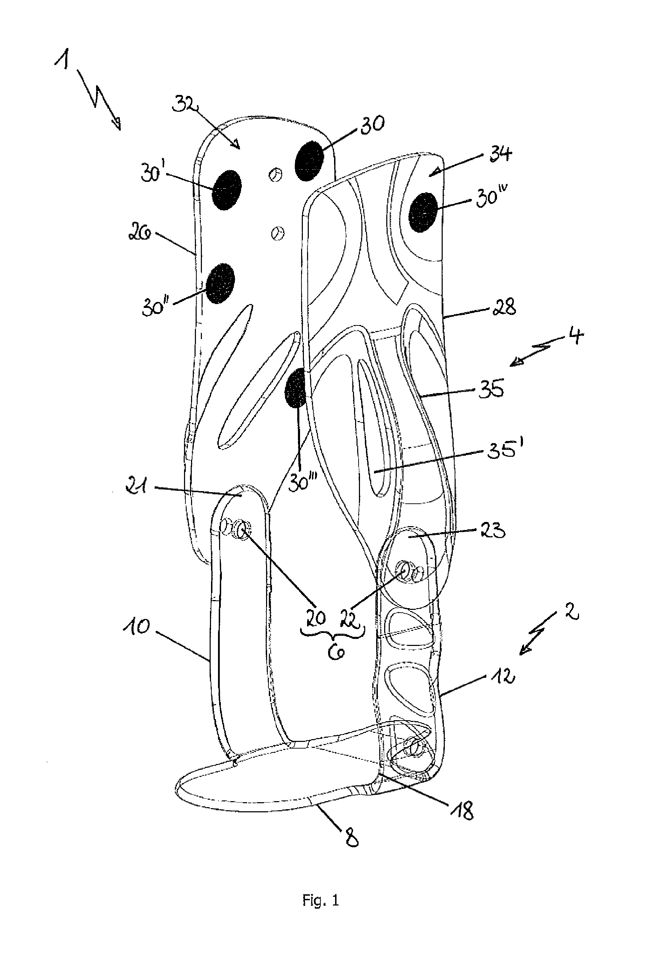

Fig. 1 zeigt eine perspektivische Darstellung einer Sprunggelenkbandage 1, die ein unteres Basiselement 2, ein Oberteil 4 und eine Gelenkverbindung 6 aufweist, die das untere Basiselement 2 mit dem Oberteil 4 verbindet. Das untere Basiselement 2 umfasst ein Bodenelement 8, ein inneres aufragendes Stützelement 10 und ein äußeres aufragendes Stützelement 12, wobei das innere aufragende Stützelement 10 an der Innenseite des Fußes anliegt und das äußere aufragende Stützelement 12 an der Außenkante des Fußes anliegt.

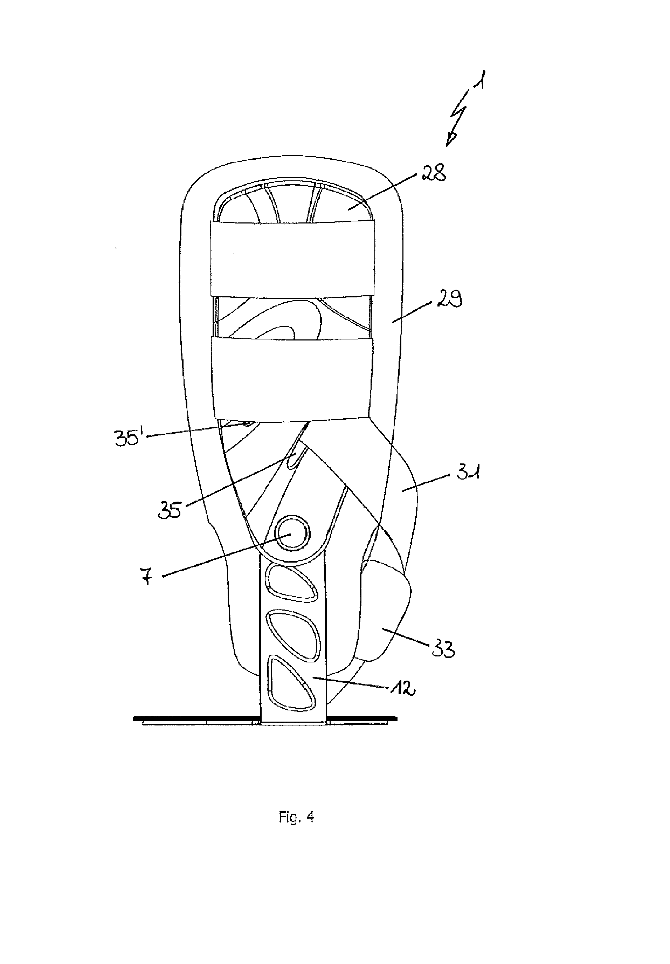

Dieses untere Basiselement 2 ist in dieser Ausführungsform als einstückiges Spritzgussteil ausgebildet. Das Oberteil 4 umfasst ein erstes schwenkbares Schaftelement 26 und ein zweites schwenkbares Schaftelement 28, die über die Gelenkverbindung 6 mit dem inneren aufragenden Stützelement 10 beziehungsweise mit dem äußeren aufragenden Stützelement 12 in Wirkverbindung stehen. Das erste schwenkbare Schaftelement 26 ist dabei über ein erstes Gelenk 20 am oberen Ende 21 des inneren aufragenden Stützelements 10 mit diesem verbunden und das zweite schwenkbare Schaftelement 28 ist mit einem zweiten Gelenk 22 am oberen Ende 23 des äußeren aufragenden Stützelements 12 mit diesem verbunden. Die Gelenkverbindung 6 ist hier vorzugsweise durch Nieten 7 realisiert (hier nicht dargestellt; siehe Fig. 4).

An den schwenkbaren Schaftelementen 26, 28 können über Befestigungselemente eine erste Polstereinlage 27 und eine zweite Polstereinlage 29 (hier nicht dargestellt; siehe Fig. 4) angebracht werden, die den Tragekomfort erhöhen und somit das Bein des Patienten vor Druckstellen oder dergleichen schonen. Diese Befestigungselemente sind Klettverschlusselemente 30. Diese Klettverschlusselemente 30 werden in einer Vielzahl an der Innenseite 32 des ersten schwenkbaren Schaftelements 26 und des zweiten schwenkbaren Schaftelements 28 angebracht und auf der Außenseite der jeweils dazugehörigen ersten Polstereinlage 27 und zweiten Polstereinlage 29 angebracht (hier nicht dargestellt).

Die Sprunggelenkbandage 1 wird über ein durch Öffnungen 35 an den schwenkbaren Schaftelementen 26, 28 geführtes Band 31 (siehe Fig. 4) am Bein des Patienten angebracht und liegt so beim Tragen der Sprunggelenkbandage 1 mit der ersten Polstereinlage 27 des ersten schwenkbaren Schaftelements 26 und der zweiten Polstereinlage 29 des zweiten schwenkbaren Schaftelements 28 an der Wade des Patienten an (hier nicht dargestellt). Dieses Band 31 ist in dieser Ausführungsform mit Klettverschlusselementen 30 an der Außenseite 34 des ersten schwenkbaren Schaftelements 26 und des zweiten schwenkbaren Schaftelements 28 befestigt. Dies garantiert einen sicheren und angenehm tragbaren Halt der Sprunggelenkbandage 1 und gewährleistet so eine Stabilität des verletzten Sprunggelenks.

Die Bodenplatte 8 wird je nachdem, welches Bein verletzt ist, mit einem Vorsprung 9 versehen, der die Auflage für die mediale Fußwölbung des Patienten bildet. Diese Bodenplatte 8 wird dann in den Schuh eingelegt und kann dann je nach Belieben und Behandlungsdauer in den Schuh eingelegt werden.

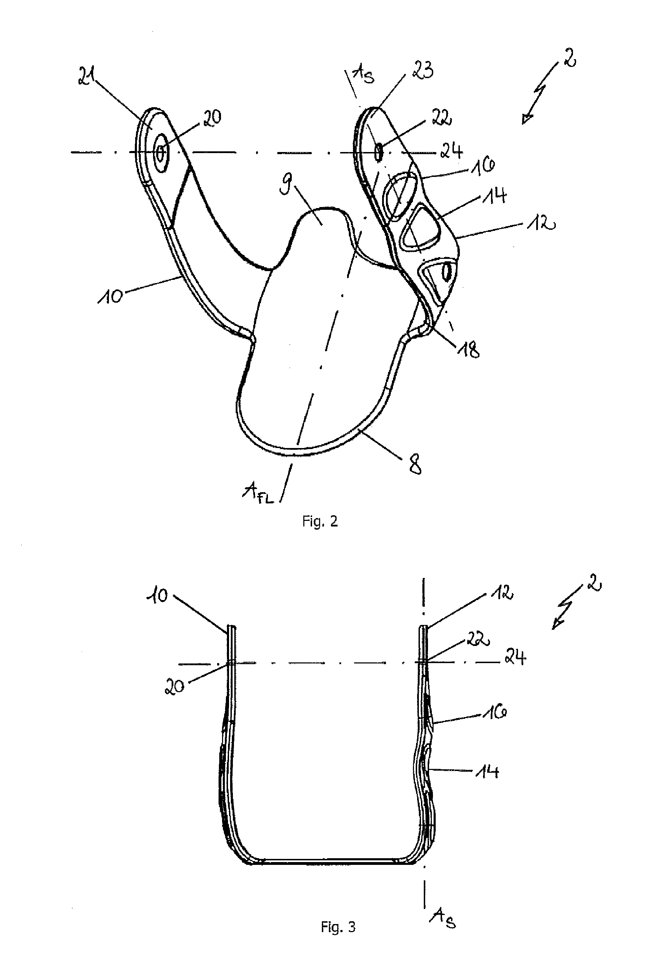

In Fig. 2 ist das untere Basiselement 2 der Sprunggelenkbandage 1 aus Fig. 1 in einer perspektivischen Darstellung gezeigt. Das einstückig ausgebildete Spritzgussteil weist das Bodenelement 8, das innere aufragende Stützelement 10 und das äußere aufragende Stützelement 12 auf. Am oberen Ende 21 des inneren aufragenden Stützelements 10 ist ein erstes Gelenk 20 angebracht.

Die Fußlängsachse A_{FL} verläuft entlang der Bodenplatte 8 horizontal in der Mitte des Fußes. Das äußere aufragende Stützelement 12 verläuft entlang einer parallel zu dieser Fußlängsachse A_{FL} verschobenen Achse, die hier zur Vereinfachung nicht dargestellt ist. Eine Senkrechte A_{S} zu dieser parallel verschobenen Achse ist die Längsachse des äußeren aufragenden Stützelements 12. Dieses äußere aufragende Stützelement 12 ist gedanklich in einen unteren Abschnitt 14 und in einen oberen Abschnitt 16 unterteilt. Dabei wird der untere Abschnitt 14 des äußeren aufragenden Stützelements 12, der unterhalb des Außenknöchels liegt, um einen Winkel α um diese Senkrechte A_{S} gedreht. Dies erfolgt in distaler Richtung nach außen, also vom Außenknöchel des Fußes weg. Der obere Abschnitt 16 des äußeren aufragenden Stützelements 12 ist wiederum in die Gegenrichtung des unteren Abschnitts 14 zurückgedreht, sodass er wieder parallel zur Fußlängsachse A_{FL} verläuft. Dies hat den Vorteil, dass das äußere aufragende Stützelement 12 eine perfekte Passform bietet und so eine physiologisch korrekte Anpassung an den Außenknöchel darstellt. Diese Passform wird durch die Ausbildung einer Auswölbung 18 verstärkt, die distal nach außen gerichtet vorgesehen ist. Beim Auftreten des Fußes unter einer Gehbewegung wird der Mittelfuß gespreizt, was bedeutet, dass sich die Breite der Fußsohle (planta pedis) vergrößert. Dadurch wird ein bequemes Tragen gewährleistet. Der Mittelfuß hat die Möglichkeit, sich auszubreiten und sich in diese Auswölbung 18 einzupassen, was zur zusätzlichen Stabilisierung des Knöchels beiträgt.

Am oberen Ende 23 des äußeren aufragenden Stützelements 12 befindet sich ein zweites Gelenk 22. Es bildet zusammen mit dem ersten Gelenk 20 des inneren aufragenden Stützelements 10 eine Gelenkverbindung 6. Über diese Gelenkverbindung 6 steht das untere Basiselement 2 mit dem Oberteil 4 der Sprunggelenkbandage 1 in Wirkverbindung (siehe Fig. 1). Dies geschieht beispielsweise über eine Verbindung mittels Nieten 7 oder dergleichen bewegliche Befestigungsverbindung (hier nicht dargestellt; siehe Fig. 4) und ermöglicht ein Schwenken des Oberteils 4 gegenüber dem unteren Basiselement 2 in Richtung der Fußlängsachse A_{FL}.

Durch das erste Gelenk 20 und das zweite Gelenk 22 verläuft eine Achse 24, die horizontal oder nahezu horizontal ist. Dies stellt die anatomisch richtig liegende Anordnung der Drehgelenke dar und fördert so durch eine parallele Anordnung eine physiologisch korrekte Fußbewegung. Somit wird eine zusätzliche Belastung des Knöchels durch eine falsche Fußbewegung verhindert.

In Fig. 3 ist das untere Basiselement 2 aus Fig. 2 in einer Frontansicht dargestellt. Hier ist die horizontale Lage der Achse 24 durch das erste Gelenk 20 und das zweite Gelenk 22 verdeutlicht, die für die physiologisch korrekte Passform der Sprunggelenkbandage 1 steht.

Auch die Außendrehung des unteren Abschnitts 14 und die reversierende Innendrehung des oberen Abschnitts 16 des äußeren aufragenden Stützelements 12 ist noch einmal verdeutlicht.

Fig. 4 zeigt eine Seitenansicht einer funktionsfähigen Sprunggelenkbandage 1 aus Fig. 1. Hier ist die Gelenkverbindung 6 zwischen dem unteren Basiselement 2 und dem Oberteil 4 durch einen Niet 7 vervollständigt. Außerdem ist die zweite Polstereinlage 29 über Klettverschlusselemente 30 (hier nicht dargestellt; siehe Fig. 1) am zweiten schwenkbaren Schaftelement 28 befestigt.

Weiterhin ist das Band 31 zu sehen, über das die Sprunggelenkbandage 1 am Fuß und insbesondere an der Wade des Patienten angebracht wird. Dieses Band 31 verläuft erst um die schwenkbaren Schaftelemente 26, 28 herum, wird durch eine Öffnung 35 in diesen schwenkbaren Schaftelementen 26, 28 hindurchgeführt und verläuft weiterhin schräg über den Vorderfuß. Dieser schräge Verlauf des Bandes 31 um den Vorderfuß beinhaltet ein zusätzliches Polster 33, das ein angenehmes Tragen der Sprunggelenkbandage 1 garantiert.
Außerdem verhindert der schräge Verlauf des Bandes 31 einen Talusvorschub, das heißt, einer Verlagerung des Sprungbeins im Sprunggelenk wird dadurch vorgebeugt und auch das vordere, äußere Band wird dadurch am Sprunggelenk gesichert.

An dieser Stelle sei darauf hingewiesen, dass alle oben beschriebenen Teile für sich alleine gesehen und in jeder Kombination, insbesondere die in den Zeichnungen dargestellten Details als erfindungswesentlich beansprucht werden. Abänderungen hiervon sind dem Fachmann geläufig.

### Bezugszeichenliste

- 1: Sprunggelenkbandage
- 2: Unteres Basiselement
- 4: Oberteil
- 6: Gelenkverbindung
- 7: Niet
- 8: Bodenelement
- 9: Vorsprung
- 10: Inneres aufragendes Stützelement
- 12: Äußeres aufragendes Stützelement
- 14: Unterer Abschnitt
- 16: Oberer Abschnitt
- 18: Auswölbung
- 20: Erstes Gelenk
- 21: Oberes Ende
- 22: Zweites Gelenk
- 23: Oberes Ende
- 24: Achse
- 26: Erstes schwenkbares Schaftelement
- 27: Erste Polstereinlage
- 28: Zweites schwenkbares Schaftelement
- 29: Zweite Polstereinlage
- 30: Klettverschlusselemente
- 31: Band
- 32: Innenseite
- 33: Polster
- 34: Außenseite
- 35: Öffnung
- α: Winkel
- A_{FL}: Fußlängsachse
- A_{S}: Senkrechte

## Patentansprüche

1. Sprunggelenkbandage zur Behandlung von Verletzungen des Sprunggelenks, mit einem unteren Basiselement (2) und einem Oberteil (4), welches über eine Gelenkverbindung (6) mit dem unteren Basiselement (2) in Wirkverbindung steht, wobei das untere Basiselement (2) ein Bodenelement (8), ein inneres aufragendes Stützelement (10) und ein äußeres aufragendes Stützelement (12) umfasst, welche miteinander verbunden sind, und wobei das äußere aufragende Stützelement (12) einen unteren Abschnitt (14) aufweist, der sich unterhalb des Außenknöchels befindet, und einen oberen Abschnitt (16), der sich oberhalb des Außenknöchels befindet,
**dadurch gekennzeichnet, dass**
der untere Abschnitt (14) des äußeren aufragenden Stützelements (12) zur Fußlängsachse (A_{FL}) um einen Winkel (α) distal nach außen gedreht ist, und der obere Abschnitt (16) des äußeren aufragenden Stützelements (12) parallel zur Fußlängsachse (A_{FL}) angeordnet ist.

2. Sprunggelenkbandage nach Anspruch 1,
**dadurch gekennzeichnet, dass**
das Bodenelement (8), das innere aufragende Stützelement (10) und das äußere aufragende Stützelement (12) einstückig miteinander verbunden sind.

3. Sprunggelenkbandage nach Anspruch 1 oder 2,
**dadurch gekennzeichnet, dass**
der untere Abschnitt (14) des äußeren aufragenden Stützelements (12) eine distal nach außen gerichtete Auswölbung (18) aufweist.

4. Sprunggelenkbandage nach einem der vorhergehenden Ansprüche,
**dadurch gekennzeichnet, dass**
die Gelenkverbindung (6) ein erstes Gelenk (20) am oberen Ende (21) des inneren aufragenden Stützelements (10) und ein zweites Gelenk (22) am oberen Ende (23) des äußeren aufragenden Stützelements (12) aufweist.

5. Sprunggelenkbandage nach einem der vorhergehenden Ansprüche,
**dadurch gekennzeichnet, dass**
die durch das erste Gelenk (20) und das zweite Gelenk (22) verlaufende Achse (24) horizontal oder nahezu horizontal verläuft.

6. Sprunggelenkbandage nach einem der vorhergehenden Ansprüche,
**dadurch gekennzeichnet, dass**
der Winkel (α) in einem Bereich von 3,5° bis 7,0° liegt.

7. Sprunggelenkbandage nach einem der vorhergehenden Ansprüche, insbesondere Anspruch 6,
**dadurch gekennzeichnet, dass**
der Winkel (α) 5,2° beträgt.
